(19) Europäisches Patentamt
European Patent Office
Office européen des brevets

(11) Veröffentlichungsnummer: **0 425 974 B1**

(12) **EUROPÄISCHE PATENTSCHRIFT**

(45) Veröffentlichungstag der Patentschrift: **01.06.94**

(51) Int. Cl.[5]: **C07C 49/84**, C07C 45/71, C07C 45/78, C07C 45/79

(21) Anmeldenummer: **90120265.5**

(22) Anmeldetag: **23.10.90**

# (54) Verfahren zur Herstellung und Reinigung von 2-Hydroxy-4-(2'-hydroxy-ethoxy)-phenylarylketonen.

(30) Priorität: **03.11.89 DE 3936592**

(43) Veröffentlichungstag der Anmeldung:
**08.05.91 Patentblatt 91/19**

(45) Bekanntmachung des Hinweises auf die Patenterteilung:
**01.06.94 Patentblatt 94/22**

(84) Benannte Vertragsstaaten:
**BE CH DE ES FR GB IT LI NL**

(56) Entgegenhaltungen:
**EP-A- 0 037 353**
**EP-A- 0 072 475**
**GB-A- 1 186 818**

(73) Patentinhaber: **BASF Aktiengesellschaft**
**Carl-Bosch-Strasse 38**
**D-67063 Ludwigshafen(DE)**

(72) Erfinder: **Aumueller, Alexander, Dr.**
**An der Marlach 5**
**W-6705 Deidesheim(DE)**
Erfinder: **Goetze, Wolfgang**
**Sohlstrasse 45**
**W-6701 Maxdorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 425 974 B1

**Beschreibung**

Die vorliegende Erfindung betrifft ein verbessertes Verfahren zur Herstellung von 2-Hydroxy-4-(2'-hydroxyethoxy)-phenylarylketonen der allgemeinen Formel I

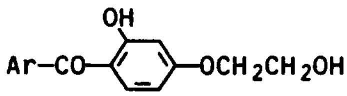 (I) ,

in der Ar für eine unsubstituierte oder substituierte Arylgruppe steht, durch Umsetzung eines 2-4-Dihydroxyphenylarylketons der allgemeinen Formel II

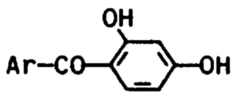 (II)

mit Ethylencarbonat in Gegenwart eines Katalysators. Weiterhin betrifft die Erfindung die Reinigung der Verbindungen I, wie sie bei technischen Synthesen als Rohprodukte anfallen.

Die Herstellung der als Lichtschutzmittel für Kunststoffe und Lacke wichtigen Verbindungen I durch Umsetzung der Verbindungen II mit Ethylencarbonat (1,3-Dioxolan-2-on) mit Hilfe von Katalysatoren ist aus verschiedenen Veröffentlichungen bekannt, wobei als Katalysatoren quartäre Ammoniumsalze (US-A-4 885 396), Alkalimetallhalogenide (US-A-4 341 905) und basische Alkalimetall- oder Erdalkalimetallcarbonate oder - alkoholate empfohlen werden (GB-A-1 186 818). Diese Katalysatoren lassen jedoch hinsichtlich der Ausbeute an den Ethoxylierungsprodukten sowie deren Reinheit zu wünschen übrig, so daß in aller Regel aufwendige Reinigungsschritte erforderlich sind."

Der Erfindung lag daher ein verbessertes Verfahren zur Herstellung der Verbindung I sowie, unabhängig vom Herstellungsverfahren, zu deren Reinigung als Aufgabe zugrunde.

Demgemäß wurde ein Verfahren zur Herstellung von 2-Hydroxy-4-(2'-hydroxyethoxy)-phenylarylketonen der allgemeinen Formel I

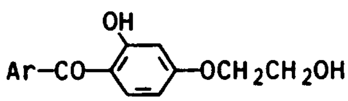 (I) ,

in der Ar für eine unsubstituierte oder substituierte Arylgruppe steht, durch Umsetzung eines 2-4-Dihydroxyphenylarylketons der allgemeinen Formel II

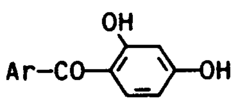 (II)

mit Ethylencarbonat in Gegenwart eines Katalysators gefunden, welches dadurch gekennzeichnet ist, daß man hierzu als Katalysator ein Alkalimetall- oder Erdalkalimetallsalz einer Carbonsäure verwendet.

Weiterhin wurde ein Verfahren zur Reindarstellung von 2-Hydroxy-4-(2' -hydroxyethoxy)-phenylarylketonen der allgemeinen Formel I gefunden, welches dadurch gekennzeichnet ist, daß man eine Lösung des Rohproduktes der Formel I mit Natriumdithionit behandelt.

Als Katalysatoren eignen sich prinzipiell die Alkalimetall- und Erdalkalimetallsalze beliebiger Carbonsäuren, wobei im Falle mehrwertiger Carbonsäuren sämtliche Säurefunktionen in Salzform vorliegen sollen. Bevorzugte Verbindungen sind die Natrium- und Kaliumslaze von Carbonsäuren wie aliphatische Carbonsäuren mit 1 bis 8 C-Atomen, z.B. Ameisen-, Essig-, Propion-, Butter-, Valerian-, Capron-, Capryl- und Caprinsäure, ungesättigte Fettsäuren, z.B. Stearin- und Ölsäure, aromatische Carbonsäuren, z.B. Benzoesäure oder Methylbenzoesäure, araliphatische Carbonsäuren, z.B. Phenylessigsäure und Hydroxycarbonsäuren, z.B. Zitronen- oder Weinsäure. Besonders gute Ergebnisse erzielt man mit dem Trinatrium- und dem Trikaliumsalz der Nitriloessigsäure und dem Tetranatrium- und dem Tetrakaliumsalz der Ethylendiamintetraessigsäure.

Die Menge des Katalysators ist nicht kritisch, beträgt im allgemeinen aber pro Säurefunktion 0,001 bis 0,1 mol-äq pro mol II, vorzugsweise 0,02 bis 0,05 mol-äq. Größere Mengen, etwa 0,25 mol-äq, bringen in der Regel keine Vorteile mehr.

Als Lösungsmittel eignen sich für die Umsetzung gegenüber den Reaktanden inerte Lösungsmittel, z.B. Ether wie Diethylenglykoldimethylether oder Anisol. Es empfiehlt sich jedoch, die Reaktion lösungsmittelfrei durchzuführen.

Vorzugsweise nimmt man die Umsetzung bei 100 bis 210 °C vor, insbesondere bei 120 bis 200 °C. Zur Vermeidung von Nebenreaktionen und zur Erzielung einer befriedigenden Reaktionsgeschwindigkeit empfiehlt sich besonders der Temperaturbereich von 140 bis 175 °C.

In aller Regel empfiehlt es sich, das Ethylencarbonat in äquimolaren Mengen oder in leichtem Unterschuß, bevorzugt jedoch in leichtem überschuß über die Ausgangsverbindung II einzusetzen. Im allgemeinen verwendet man pro mol 2,4-Dihydroxyphenylarylketon 0,95 bis 1,5 mol Ethylencarbonat. Größere Mengen sind möglich, bringen aber in der Regel keine weiteren Vorteile mehr. Die Reaktionszeiten liegen im allgemeinen zwischen 2 und 12 Stunden.

Die Aufarbeitung bietet methodisch keine Besonderheiten. Sie kann destillativ oder durch Kristallisation erfolgen.

Als Ausgangsverbindungen II kommen prinzipell alle 2,4-Dihydroxyphenylarylketone in Betracht. Im Hinblick auf die Eignung der Verfahrensprodukte I als Lichtschutzmittel werden diejenigen Ausgangsstoffe II bevorzugt, in denen der Arylrest eine Phenylgruppe bedeutet, welche mit Halogen wie Fluor, Chlor und Brom, mit Cyan- und Hydroxygruppen, mit $C_1$- bis $C_8$-Alkylgruppen, z.B. Methyl-, Ethyl-, Propyl-, Isopropyl- und Butylgruppen, mit $C_1$- bis $C_8$-Alkoxygruppen, z.B. Methoxy-, Ethoxy- und Propoxygruppen oder mit Phenyl-, Phenoxy- und Benzylgruppen substituiert ist. Bevorzugt sind para-substituierte Phenyl- und o-Hydroxyphenylgruppen. Besonders wichtige Verbindungen 1 bzw. II sind solche, in denen Ar eine unsubstituierte Phenylgruppe oder ein p-Chlor-, p-Methyl-, p-Methoxy-, p-Ethyl-, p-Ethoxy-, o-Hydroxy- oder o-Hydroxy-p-(2-hydroxyethoxy)-phenylgruppe bedeutet.

Die als Ausgangsstoffe eingesetzten 2,4-Dihydroxyphenylarylketone sind bekannt und können auf übliche Art und Weise, z.B. durch Friedels-Craft-Acylierung von Resorcin mit Carbonsäurechloriden erhalten werden.

Unabhängig vom Herstellungsverfahren wurde ein neues Verfahren zur Reinigung von 2-Hydroxy-4-(2'-hydroxyethoxy)-phenylarylketonen entwickelt. Das Reinigungsverfahren ist von der Provenienz der Produkte nicht abhängig.

Dazu werden die roh anfallenden 2-Hydroxy-4-(2'-hydroxyethoxy)-phenylarylketone mit Natriumdithionit in Wasser, einem $C_1$- bis $C_6$-Alkohol oder einem Gemisch dieser Lösungsmittel, gegebenenfalls in Gegenwart einer Protonensäure und Aktivkohle, versetzt.

Das Natriumdithionit kommt in Mengen von 0,5 bis 15 Gew.%, insbesondere 5 bis 10 Gew.%, bezogen auf das Rohprodukt, zum Einsatz.

Als Lösungsmittel eignen sich Wasser, gesättigte $C_1$- bis $C_6$-Alkohole, dabei bevorzugt Propanol, iso-Propanol, n-Butanol, iso-Butanol, n-Anylalkohol, tert.-Amylalkohol, n-Hexanol und besonders bevorzugt Methanol und Ethanol sowie Gemische der Lösungsmittel.

Im allgemeinen empfiehlt sich, das Rohprodukt I in einem 2- bis 5-fachen überschuß des Lösungsmittels 0,25 bis 3 h mit dem Natriumdithionit zu kochen. Gegebenenfalls wird eine Protonensäure, insbesondere Schwefelsäure, vorzugsweise 10 bis 20 Gew.%, bezogen auf den Alkohol, und/oder Aktivkohle, vorzugsweise 0 bis 20 Gew.%, insbesondere 5 bis 10 Gew.% zugesetzt. Das Reinprodukt I wird über Auskristallisation wie üblich aufgearbeitet, so daß sich weitere Angaben erübrigen.

Die Verbindungen I, die nach dem Reinigungsverfahren hochrein erhalten werden, finden als UV-Absorber in Kunststoff und Lacken Verwendung, vorzugsweise dort, wo die Eigenfarbe des Zusatzes störend wirkt wie z.B. in Polystyrol und ABS, insbesondere in glasklarem Polycarbonat.

Beispiele

Herstellung von 2-Hydroxy-4-(2'-hydroxyethoxy)-phenylarylketonen

Beispiele 1 bis 8

418 g (2 mol) 2,4-Dihydroxybenzophenon wurden mit 193 g (2,2 mol) Ethylencarbonat und 0,02 bis 0,2 mol Katalysator versetzt und solange auf 155 °C erhitzt, bis keine Gasentwicklung mehr stattfand. Die Aufarbeitung des flüssigen Produktes erfolgte wie üblich über Kristallisation und Destillation. Die Einzelheiten und die Ergebnisse dieser Versuche sind der Tabelle 1 zu entnehmen.

Tabelle 1

| Katalysator Menge [mol] | Umsatz % | Reaktionsdauer/h | Ausbeute % | Reinheit % |
|---|---|---|---|---|
| Erfindungsgemäß | | | | |
| 1. Ethylendiamintetraessigsäuretetranatriumsalz (0,02) | 99,5 | 7 | 95 | 97,5 |
| 2. Trikaliumcitratmonohydrat (0,02) | 99,7 | 7 | 96 | 97,6 |
| 3. Nitriloessigsäuretrinatriumsalz (0,02) | 99,4 | 8 | 95 | 97,8 |
| 4. Natriumoleat (0,05) | 99,9 | 7 | 95 | 98,2 |
| 5. Natriumstearat (0,05) | 99,6 | 7 | 95 | 98,5 |
| Vergleichsversuche | | | | |
| 6. Tetrabutylammoniumjodid (0,02) | 68,9 | 8 | 92 | 96,8 |
| 7. Kaliumjodid (0,02) | 89,1 | 8 | 96 | 92,3 |
| 8. Natriumjodid (0,02) | 92,1 | 8 | 92 | 96,8 |

Verfahren zur Reinigung von 2-Hydroxy-4-(2' -hydroxyethoxy)-phenylarylketonen

Beispiel 9

50 g des Rohproduktes I, wie es nach den Beispielen 1 bis 8 vor der Aufarbeitung anfiel, wurden mit 2,5 g Natriumdithionit, 2,5 g Aktivkohle und 10 ml 2 n Schwefelsäure zusammen in einem Gemisch von 100 ml Ethanol und 50 ml Wasser zum Rückfluß erhitzt. Nach Abfiltern ungelöster Bestandteile in der Hitze wurde die Lösung mit 50 ml Wasser versetzt und nach dem Abkühlen wurde das gereinigt Produkt abfiltriert, mit 200 ml Wasser gewaschen und getrocknet. Die Ausbeute an Reinprodukt betrug 46,7 g.

Glasklares Polycarbonat, das mit 0,2 Gew.-% des UV-Absorbers versetzt worden war, zeigte einen Wert von 9,1 nach dem Yellownes-Index (ASTM D 1925). Im Falle des rohen Absorbers betrug der Wert 11,8 und ohne Zusatz des Stoffes zum Polycarbonat 7,1.

## Patentansprüche

1. Verfahren zur Herstellung von 2-Hydroxy-4-(2' -hydroxyethoxy)-phenylarylketonen der allgemeinen Formel I

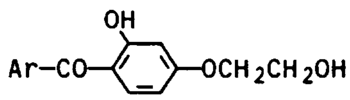

(I) ,

in der Ar für eine unsubstituierte oder substituierte Arylgruppe steht, durch Umsetzung eines 2,4-Dihydroxyphenylarylketons der allgemeinen Formel II

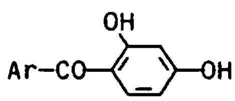

(II)

mit Ethylencarbonat in Gegenwart eines Katalysators, dadurch gekennzeichnet, daß man hierzu als Katalysator ein Alkalimetall- oder Erdalkalimetallsalz einer Carbonsäure verwendet.

2. Verfahren nach Anspruch 1, dadurch gekennzeichnet, daß man als Katalysator das Natrium- oder Kaliumsalz einer $C_1$- bis $C_{18}$-Fettsäure, das Trinatrium- oder Trikaliumsalz der Nitrilotriessigsäure oder das Tetranatrium- oder Tetrakaliumsalz der Ethylendiamintetraessigsäure verwendet.

3. Verfahren nach den Ansprüchen 1 oder 2, dadurch gekennzeichnet, daß man es auf die Herstellung solcher Verbindungen I anwendet, in denen Ar eine Phenylgruppe bedeutet, die bis zu zwei der folgenden Substituenten tragen kann:
   a) Fluor, Chlor, Brom, Cyan, Hydroxy
   b) $C_1$-$C_8$-Alkyl und -Alkoxy, wobei diese Reste ihrerseits Substituenten der Gruppe a tragen können
   c) Phenyl, Phenoxy und Benzyl, wobei diese Gruppen ihrerseits Substituenten der Gruppen a und b tragen können.

4. Verfahren zur Reindarstellung von 2-Hydroxy-4-(2' -hydroxyethoxy)-phenylarylketonen der allgemeinen Formel I gemäß Anspruch 1, dadurch gekennzeichnet, daß man eine Lösung eines Rohproduktes der Formel I mit Natriumdithionit behandelt.

5. Verfahren nach Anspruch 4, dadurch gekennzeichnet, daß man die Lösung des Rohproduktes der Formel I zusätzlich mit einer starken Mineralsäure behandelt.

6. Verfahren nach den Ansprüchen 4 oder 5, dadurch gekennzeichnet, daß man die Lösung des Rohproduktes der Formel I mit Aktivkohle versetzt.

## Claims

1. A process for preparing a 2-hydroxy-4-(2'-hydroxyethoxy)phenyl aryl ketone of the formula I

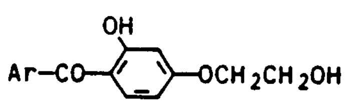

(I)

where Ar is substituted or unsubstituted aryl, by reacting a 2,4-dihydroxyphenyl aryl ketone of the formula II

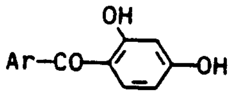

(II)

with ethylene carbonate in the presence of a catalyst, which comprises using a catalyst that is an alkali metal or alkaline earth metal salt of a carboxylic acid.

2. A process as claimed in claim 1, wherein the catalyst used is the sodium or potassium salt of a $C_1$-$C_{18}$-fatty acid, the trisodium or tripotassium salt of nitrilotriacetic acid or the tetrasodium or tetrapotassium salt of ethylenediaminetetraacetic acid.

3. A process as claimed in claim 1 or 2 for preparing a compound I where Ar is phenyl which may carry up to two of the following substituents:
   a) fluorine, chlorine, bromine, cyano, hydroxyl
   b) $C_1$-$C_8$-alkyl and $C_1$-$C_8$-alkoxy, which may each in turn be substituted by substituents of group a
   c) phenyl, phenoxy and benzyl, which may each in turn be substituted by substituents of groups a and b.

4. A process for purifying a 2-hydroxy-4-(2'-hydroxyethoxy)phenyl aryl ketone of the formula I as defined in claim 1, which comprises treating a solution of a crude product of the formula I with sodium dithionite.

5. A process as claimed in claim 4, wherein the solution of the crude product of the formula I is additionally treated with a strong mineral acid.

6. A process as claimed in claim 4 or 5, wherein the solution of the crude product of the formula I is treated with activated carbon.

## Revendications

1. Procédé de préparation de 2-hydroxy-4-(2'-hydroxyéthoxy)-phénylarylcétones de la formule générale I

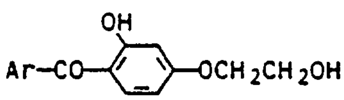

(I) ,

dans laquelle Ar est mis pour un groupe aryle substitué ou non, par réaction d'une 2,4-dihydroxy-phénylarylcétone de la formule générale II

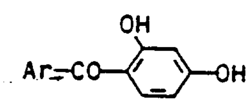

(II)

avec du carbonate d'éthylène en présence d'un catalyseur, caractérisé par le fait que l'on utilise ici comme catalyseur un sel de métal alcalin ou alcalino-terreux d'un acide carboxylique.

2. Procédé selon la revendication 1, caractérisé par le fait que l'on utilise, comme catalyseur, le sel de sodium ou de potassium d'un acide gras en C1 à C18, le sel de trisodium ou tripotasssium de l'acide nitrilotriacétique ou le sel de tétrasodium ou tétrapotassium de l'acide éthyléndiaminotétracétique.

3. Procédé selon la revendication 2, caractérisé par le fait qu'il s'applique à la préparation de composés I dans lesquels Ar représente un groupe phényle qui peut porter jusqu'à deux des substituants suivants :
   a) fluor, chlore, brome, cyano, hydroxy
   b) alkyle et alcoxy en C1-C8, ces restes pouvant, à leur tour, porter des substituants du groupe a)

c) phényle, phénoxy et benzyle, ces groupes, à leur tour, pouvant porter des substituants des groupes a et b.

**4.** Procédé de préparation à l'état pur de 2-hydroxy-4-(2'-hydroxyéthoxy)-phénylarylcétones de la formule I selon la revendication 1, caractérisé par le fait que l'on traite une solution d'un produit brut de formule I avec du dithionite de sodium.

**5.** Procédé selon la revendication 4, caractérisé par le fait que l'on traite la solution du produit brut de formule I additionnellement avec un acide minéral fort.

**6.** Procédé selon la revendication 4 ou 5, caractérisé par le fait que l'on mélange la solution du produit brut de formule I avec du charbon actif.